# EUROPEAN PATENT SPECIFICATION

(11) **EP 0 435 671 B1**
(45) Date of publication and mention of the grant of the patent: **28.09.1994**
(21) Application number: 90314329.5
(22) Date of filing: 27.12.1990
(51) Int. Cl.: A23K 1/16, A23K 1/18, A61K 35/413

(54) **Disease resistance ration and feed additive for cattles and swines**
Krankheitsresistenz gebende Rationen und Futtermittelzusatz für Vieh und Schweine
Rations donnant de la résistance aux maladies et additif alimentaire pour bétail et porcs

(30) Priority: 27.12.1989 JP 340728/89
(43) Date of publication of application: 03.07.1991
(73) Proprietor: NATIONAL FEDERATION OF AGRICULTURAL CO-OPERATIVE ASSOCIATIONS, Tokyo (JP)
(72) Inventor: Hamano, Atsushi, Ibaraki-ken (JP); Ogawa, Megumi, Sakura-shi, Chiba-ken (JP); Sasaki, Takashi, Sakura-shi, Chiba-ken (JP)
(74) Representative: Cresswell, Thomas Anthony

(56) References cited:
- PATENT ABSTRACTS OF JAPAN, vol. 4, no. 81 (C-14)[563], 11th June 1980;& JP-A-55 45 314 (SANWA K.K.) 31-03-1980
- PATENT ABSTRACTS OF JAPAN, vol. 13, no. 472 (C-647)[3820], 25th October 1989;& JP-A-1 186 817 (TOKYO TANABE CO., LTD) 26-07-1989
- DERWENT FILE SUPPLIER WPIL, 1987, accession no. 87-217588 [31], Derwent
- Publications Ltd, London, GB;& JP-A-62 143 647 (TANABE SEIYAKU K.K.) 26-06-1987
- DERWENT FILE SUPPLIER, WPI, 1971, accession no. 71-66099S [41], DerwentPublications Ltd, London, GB;& JP-B-71 035 390 (S. OKIBAYASHI)
- DERWENT FILE SUPPLIER, WPI, 1966, accession no. 66-37691F [00], DerwentPublications Ltd, London, GB;& SU-A-214 030 (NG BELEN KII)
- DERWENT FILE SUPPLIER WPIL, 1982, accession no. 82-91261E [43], DerwentPublications Ltd, London, GB;& JP-A-57 150 351 (NISSAN GOSEI KOGYO) 17-09-1982
- PATENT ABSTRACTS OF JAPAN, vol. 10, no. 139 (C-348)[2196], 22nd May 1986;& JP-A-61 1356 (NISSHIN SEIFUN K.K.) 07-01-1986

## Description

The present invention relates to a feed for cattle or swine containing safe natural materials having a weight gain effect and an improving effect on the clinical condition of livestock.

There are many problems to be solved in the field of livestock breeding, one of which is a financial loss caused by various diseases of livestock due to a poor sanitary environment. As countermeasures against livestock diseases specially caused by microbes, various additives have been used in feed, such as synthetic antimicrobial or antibiotic substances, and livestock have been vaccinated, but these measures have not been working efficiently against various infectious diseases of livestock. Excessive medication sometimes causes a reduction in productivity due to unexpected side effects of the additives. Also the commercial value of livestock products can be adversely affected by residual medicines therein. Accordingly, there has been required some safe and labor-saving disease preventing measure.

It is almost impossible to prevent all diseases in livestock farms by using vaccination and antibiotic medicines such as antimicrobial substances. If it could have been done, it might have required a large amount of expense and medicine. Also, there is the problem to be resolved of side effects of medicines and residual medicines in the products, when medicines are used widely.

The present invention has been developed considering the above situation and provides a safe disease resistance feed permitting an improvement in the clinical condition of cattle and swine and a high weight gain therein.

According to the present invention there is provided a feed for cattle or swine containing from 0.05 to 0.2% by weight of bile powder.

The feed of the invention can also contain at least one other safe natural substance such as licorice (Glycyrrhizae Rodix), licorice extracts, garlic powder (powder of allium scorodoprasum L. var. viviparum Regal) or quillaja extract. Feeds in accordance with the invention can improve the clinical condition of cattle and swine in a livestock farm contaminated with infectious microbes, resulting in an efficient growth of livestock therein.

The bile powder can be obtained by directly pulverizing a bile element contained in cattle and swine gall bladders optionally after extracting them by an alcoholic extracting method. The principal ingredients contained in the bile powder are cholic acid and deoxycholic acid. About 50% of cholic acids is contained in the bile powder.

Various refined products of cholic acid, contained in a bile powder composition, have been used as medicine for human gall and as a secretory accelerator of digestive enzymes and a digestive fluid, and their safety is well recognized.

Licorice (Glycyrrhizae Rodix) is a plant of the pulse family, of which root is dried to be crushed or pulverized, and an extract thereof is dark brown and adhesive, having a particularly sweet and bitter flavour generally. However, its color varies according to an extracting process thereof. Its main composition is a glycyrrhetic acid having a natural killer activity and interferon inductivity, of which effect against human simplex herpes virus is widely recognized. The licorice powder and an extract thereof absorbed in other feeds are added to livestock feed.

Garlic powder is made from garlic (Allium Scorodoprasum L. var. viviparum Regal) by drying and pulverizing scales thereof. Garlic powder contains a glycoside of glycominar. When it is hydrolyzed, a kind of volatile oil containing sulfur and diaryldisulphide, an aromatic substance, is obtained. Garlic powder also contains allysine and has a physiological activity such as an improvement of intestinal absorption of vitamin B₁.

Quillaja extract is a white to light yellowish brown powder or brown to dark brown pasty substance having a surface activity and has been used as a detergent and foaming agent for a long time. The main composition of the quillaja extract is an oligoglycoside such as triterpenoid and steriod. An extract of quillaja saponaria mol, an evergreen tree of rose family, is mainly used to get the quillaja extract. When the quillaja extract is used as feed additive, the quillaja powder can be mixed in a feed directly or a pasty quillaja powder can be absorbed in a feed.

When the bile powder is used as feed additive, it is necessary first to prepare a concentrate containing more than 50% of bile powder. A normal feed must be prepared so as to contain from 0.05 to 0.2 % of bile powder by using the concentrate containing 50% of bile powder in accordance with the degree of microbial contamination of the respective livestock farm. For example, when a concentrate containing 50 % of bile powder is used, the content of bile powder in the feed will be 0.1 % when 0.2 % of concentrate is added to a feed.

It is necessary to prepare a bile powder mixture on the basis of dry weight of the bile powder and other materials such as one part of bile powder to 0.1 to 1.0 part of garlic powder, one part of bile powder to 0.001 to 0.05 part of glycyrrhetic acid value of licorice or licorice extract, and one part of bile powder to 0.001 to 0.01 part of triterpenoid (quillajasaponin) value of quillaja extract.

Also other commercial materials sold as feeds or feed additives for livestock can be used for preparing the bile powder mixture, which are selectively used considering the breed, day-old age, weight, nutrition and palatability of the livestock. It is necessary to include vitamins and minerals in the feed of course, which may be mixed or added as with other commercial materials.

The physical forms of the disease resistance feed and the feed additive may be powder, grain or liquid. The feeds are used selectively in accordance with the feeding conditions and installations of the farm.

The bile powder and grain thereof are as easy to handle as common feed because of their forms aforementioned. When they are mixed into liquid feed, there is another merit in improving the environmental condition by preventing dust rising.

The bacteriastatic effect of bile against bacteria of livestock is disclosed by the present invention. In addition, an antivirus effect of the bile powder is disclosed against a virus of Aujeszky's disease, a virus causing various diseases among livestock, against which there had been no cure. The antivirus effect thereof is more than the same of cholic acids extracted and refined from bile powder. Therefore, it is more useful economically to use the bile powder directly in a feed than to use the cholic acid as an extracted and refined substance.

The bile powder works directly on microbes such as virus and bacteria. Licorice, garlic powder, quillaja powder or their extracts activate to fortify physiological functions and cells of live bodies, especially immunocytes, and contribute to an improvement in clinical condition and weight gain effect of cattle and swine indirectly.

The disease resistance feed of the present invention is composed of safe natural substances such as bile powder, licorice, garlic powder and quillaja powder. Therefore, the feed is harmless to human and animals and contributes to an improvement in clinical condition of cattle and swine and improves the weight gain effect.

Hereinafter, the present invention will be described in detail referring to some embodiments as follows.

### Embodiment No. 1

Bile powder prepared from freezing and drying the bile of cattle and swine was resolved in PBS (phosphate buffer saline solution) and sterilized through a milipore filter of 0.45 »m. Then, the content of the bile powder in solution was adjusted to 0, 0.03, 0.06, 0.125, 0.25, 0.5, and 1.0%. The bile-PBSs were mixed equally with Aujeszky's disease virus solution (8.5 x 10⁸ PFU/ml) of swine previously prepared and incubated at 37°C for 60 minutes. Then, the mixture was diluted 100 times in MEM (Eagle's minimum essential medium) solution. Then it was applied on CPK (cell of piglet kidney) and its effect was evaluated after 5 days. The content, at which CPE (cytopathic effect) was not observed, was evaluated as effective.

The results of the evaluation are shown in Table 1 below.

**Table 1**

| Antivirus Effect of the Bile Powder | | | | | | | |
|---|---|---|---|---|---|---|---|
| | content of bile powder | | | | | | |
| | 0 | 0.03 | 0.06 | 0.125 | 0.25 | 0.5 | 1(%) |
| bile powder of cattle | + | + | + | - | - | - | - |
| bile powder of swine | + | + | + | - | - | - | - |
| where + : CPE evaluated (ineffective) - : CPE not evaluated (effective) | | | | | | | |

The antivirus effect of the bile powder prepared from cattle and swine biles was confirmed.

### Embodiment No. 2

Bile powder prepared from freezing and drying the bile of cattle and swine was dissolved in PBS (phosphate buffer saline) and filtered through a milipore filter of 0.45 »m. Then, the content of the cholic acid in solution was adjusted to 0, 0.0125, 0.025, 0.05, 0.1, 0.2, and 0.4%, based on the total quantity of the cholic acid.

The bile-PBS's adjusted on the cholic acid quantity, were mixed equally with Aujeszky's virus solution (8.5 x 10⁸ PFU/ml) previously prepared and incubated at 37°C for 60 minutes. Then, the mixture was diluted 100 times in MEM solution. Then, it was applied to CPK (cell of piglet kidney) and its effect was evaluated after 5 days. In the same manner, the values of deoxycholic acid and sodium cholic acid were adjusted and the effect was compared with the effect of the bile powder.

The content, at which CPE (cytopathic effect) was not observed was evaluated as effective.

The results of the evaluation are shown in Table 2 below.

**Table 2**

| Comparison between the antivirus effects of the bile powder and the cholic acids | | | | | | |
|---|---|---|---|---|---|---|
| | 0 | 0.0125 | 0.025 | 0.05 | 0.1 | 0.4 (%) |
| bile powder of swine | + | + | + | - | - | - |
| bile powder of cattle | + | + | + | - | - | - |
| sodium deoxycholic acid | + | + | + | - | - | - |
| sodium cholic acid | + | + | + | + | - | - |
| wherein + : CPE evaluated (ineffective) - : CPE not evaluated (effective) | | | | | | |

### Embodiment No. 3

Bile powder prepared from freezing and drying bile of swine was resolved in triptosoy medium solution and filtered at high temperature. Then the content of the bile powder in solution was adjusted to 0, 0.5 and 1.0%.

Staphylococcus aureus 209P solution, which had been prepared previously, was inoculated by a platinum needle on 3 ml of triptosoy medium solution containing bile powder in test tube and incubated at 37°C. Its OD (optical density) value (wave length:600 nm) was measured periodically with turbidmeter so as to evaluate growth of the microbe. The OD value was 0.8 in 0 % solution of bile powder, 0.5 in 0.5 % solution of bile powder and 0.4 in 1.0 % solution of bile powder after 10 hours' incubation. The OD value showed less microbe quantity in high content solutions of bile powder. Accordingly, a bacteriostatic effect of the bile powder against Staphylococcus aureus was confirmed.

### Embodiment No. 4

Feed additives A containing 50% of bile powder, B containing 50% of bile powder and 25% of garlic powder, C containing 50% of bile powder, 25% of garlic powder and 3% of licorice and D containing 50% of bile powder, 25% of garlic powder and 1% of quillaja extract were prepared and added to a commercial feed for swine so as to be 0.2% of content.

The resulting feeds were evaluated in a livestock farm, which used to show such poor clinical signs as cough and diarrhoea, and a low productivity. The feeds containing additives A, B, C and D were each fed to 10 young swine 15 to 20 days old for 45 days. Then, their clinical signs, faeces condition, weight gains and survival ratio were compared with those of another group of young swine fed with a common swine feed without the additives for same period. The results are shown in Table 3 below.

All of the young swine fed with feeds containing additives A to D, showed an improvement in clinical signs including diarrhoea and weight gain effect when compared with those fed with a feed not containing the additives aforementioned.

**Table 3**

| Clinical conditions and weight gains of young swine | | | | |
|---|---|---|---|---|
| test group | weight gain | clinical condition (score) | diarrhoea (score) | survival |
| control | 100 | 100 | 100 | 9/10 |
| feed with additive A | 112 | 75 | 75 | 10/10 |
| feed with additive B | 115 | 69 | 78 | 10/10 |
| feed with additive C | 114 | 65 | 78 | 10/10 |
| feed with additive D | 118 | 68 | 76 | 10/10 |
| where weight gain: average weight gain of 9 young swine from the control group is considered as a score of 100. clinical sign: each score 1 to cough, snivel and poor appetite, and averages calculated considering an average for the 9 surviving for 45 days in the control group as a score of 100. diarrhoea: each score 1 to thin or pasty faeces, and averages calculated considering an average for the 9 surviving for 45 days in the control group as a score of 100. | | | | |

## Claims

1. A feed for cattle or swine containing from 0.05 to 0.2% by weight of bile powder.

2. A feed according to claim 1 which also contains at least one of licorice, licorice extract, garlic powder and quillaja extract.

3. A feed according to claim 1 or 2 in powder, granular or liquid form.

4. A feed according to claim 1, 2 or 3 wherein the bile powder is obtainable by pulverizing the gall bladders of cattle or swine.

## Patentansprüche

1. Futter für Rinder oder Schweine, das von 0,05 bis 0,2 Gew.-% Gallenpulver enthält.

2. Futter nach Anspruch 1, welches gleichfalls mindestens einen Vertreter von Süßholz, Süßholz-Extrakt, Knoblauchpulver und Seifenbaum-Extrakt enthält.

3. Futter nach Anspruch 1 oder 2 in pulverförmiger, granulärer oder flüssiger Form.

4. Futter nach Anspruch 1, 2 oder 3, worin das Gallenpulver durch Pulverisierung der Gallenblasen von Rindern oder Schweinen gewinnbar ist.

## Revendications

1. Aliment pour bétail ou porcs contenant de 0,05 à 0,2 % en poids de poudre de bile.

2. Aliment selon la revendication 1, qui contient également au moins une substance choisie parmi de la réglisse, un extrait de réglisse, de la poudre d'ail et de l'extrait de bori de Panama.

3. Aliment selon la revendication 1 ou 2, sous forme de poudre, de granule ou de liquide.

4. Aliment selon la revendication 1, 2 ou 3, dans lequel la poudre de bile peut être obtenue en pulvérisant les vésicules biliaires de bétail ou de porcs.
